# EUROPEAN PATENT APPLICATION

(11) **EP 4 437 969 A1**
(43) Date of publication of application: **02.10.2024**
(21) Application number: 21968278.8
(22) Date of filing: 15.12.2021
(51) Int. Cl.: A61B 8/08, A61B 8/04

(54) **SHEAR WAVE PARAMETER MEASUREMENT METHOD AND ULTRASONIC WAVE DEVICE**

(30) Priority: 13.12.2021 KR 20210178118
(71) Applicant: Alpinion Medical Systems Co., Ltd., Hwaseong-si, Gyeonggi-do 18365 (KR)
(72) Inventor: YUN, Chul Hee, Anyang-si Gyeonggi-do 14112 (KR); CHANG, Sun Yeob, Seoul 03477 (KR); PARK, Sung Bae, Chuncheon-si Gangwon-do 24303 (KR)
(74) Representative: Sander, Rolf
(86) International application number: PCT/KR2021/019129
(87) International publication number: WO 2023/113069

(57) **Abstract**

Disclosed are a shear wave parameter measurement method and an ultrasound wave device. The shear wave parameter measurement method according to one embodiment comprises the steps of: acquiring a plurality of shear wave data by repeating a plurality of shear wave acquisition sequences; and measuring a mechanical parameter of the plurality of acquired shear wave data, wherein the plurality of shear wave acquisition sequences comprise a first sequence and a second sequence, the first sequence comprises an operation for performing first acoustic radiation force impulse (ARFI) pushing for generating a shear wave in an area of interest, and detecting, from a plurality of detection lines, the shear wave generated by the first ARFI pushing, and the second sequence comprises an operation for performing second ARFI pushing for generating a shear wave in an area of interest, and detecting, from the plurality of detection lines, the shear wave generated by the second ARFI pushing.

## Description

### [Technical Field]

The present invention relates to technology for providing an elastography image using a shear wave, and more specifically, to technology for measuring a parameter of a shear wave.

### [Background Art]

Point shear wave elastography (PSWE) is a method for non-invasively and non-destructively measuring the stiffness of a tissue within a predefined region of interest (ROI).

PSWE evaluates tissue stiffness non-invasively and enables the examination of biological tissues without tissue extraction. PSWE is used to diagnose liver tumors and cirrhosis, measure spleen stiffness for hypertension diagnosis, and diagnose and monitor kidney stiffness.

### [Disclosure]

### [Technical problem]

The present invention proposes a shear wave measurement method and an ultrasound wave device to enhance accuracy and validation of shear wave data.

### [Technical Solution]

A shear wave parameter measurement method according to one embodiment includes the steps of: acquiring multiple shear wave data by repeating multiple shear wave acquisition sequences, and calculating mechanical parameters of the acquired multiple shear wave data, wherein the multiple shear wave acquisition sequences include a first sequence and a second sequence, the first sequence includes operations of performing first acoustic radiation force impulse (ARFI) pushing to generate a shear wave in a region of interest and detecting, from multiple detecting lines, the shear wave generated by the first ARFI pushing, and the second sequence includes operations of performing second ARFI pushing to generate a shear wave in the region of interest and detecting, from the multiple detecting lines, the shear wave generated by the second ARFI pushing.

The calculating of the mechanical parameter of the multiple shear wave data may include measuring a particle velocity of each shear wave, estimating a lag of each shear wave, calculating a speed of each shear wave, and estimating an elastic modulus of each shear wave.

The shear wave parameter measurement method may further include the steps of superimposing multiple shear-wave speed data, determining the inlier of each shear-wave speed data for each depth when superimposed, and handling outliers.

In the determining of the inlier of each shear-wave speed data, when both first shear-wave speed data and second shear-wave speed data at the same depth are outliers, the corresponding depth may be determined to be an invalid position.

In the determining of the inlier of each shear-wave speed data, when at least one of first shear-wave speed data or second shear-wave speed data at the same depth is an inlier, the corresponding depth may be determined to be a valid position.

The shear wave parameter measurement method may further include the step of calculating a validation index using the valid position of the shear-wave speed data.

The shear wave parameter measurement method may further include the steps of measuring a signal-to-noise ratio (SNR) of each shear-wave speed data, determining an inlier of each shear-wave speed data using the SNR of each shear-wave speed energy, and handling outliers.

A shear wave parameter measurement method according to another embodiment may include the steps of: acquiring multiple shear wave data by repeating multiple shear wave acquisition sequences, and calculating a mechanical parameter of the acquired multiple shear wave data, wherein the multiple shear wave acquisition sequences may include a first sequence and a second sequence, the first sequence may include operations of performing ARFI pushing to generate a shear wave in a region of interest and detecting, from a first detecting line, the shear wave generated by the first ARFI pushing, and the second sequence may include operations of performing second ARFI pushing to generate a shear wave in a region of interest and detecting, from a second detecting line, the shear wave generated by the second ARFI pushing.

The shear wave parameter measurement method may further include the steps of superimposing multiple shear-wave speed data, determining the inlier of each shear-wave speed data for each depth when superimposed, and handling outliers.

An ultrasound wave device according to another embodiment may include an ultrasound probe configured to acquire multiple shear wave data by repeating multiple shear wave acquisition sequences, and a processor configured to calculate a mechanical parameter of the shear wave data acquired through the ultrasound probe, wherein the multiple shear wave acquisition sequences may include a first sequence and a second sequence, the first sequence may include operations of performing first ARFI pushing to generate a shear wave in a region of interest and detecting, from multiple detecting lines, the shear wave generated by the first ARFI pushing, and the second sequence may include operations of performing second ARFI pushing to generate a shear wave in a region of interest and detecting, from the multiple detecting lines, the shear wave generated by the second ARFI pushing.

### [Advantageous Effects]

According to the shear wave parameter measurement method and ultrasound wave device in accordance with an embodiment, shear-wave speed samples are obtained using the data acquired through repetition of shear wave acquisition sequences, allowing the number of shear-wave speed samples to increase at each depth, and efficient handling of outliers can be achieved by determining an inlier of data. In addition, data validity can be improved by minimizing the detecting locations of shear wave data and motion artifact variables.

### [Description of Drawings]

FIG. 1 is a diagram illustrating the flow of a conventional shear wave parameter measurement method.
FIG. 2 is a diagram illustrating the flow of a method of acquiring a shear wave parameter according to one embodiment of the present invention.
FIG. 3 is a diagram illustrating a multiple pushing + multi-line detecting method according to one embodiment of the present invention.
FIG. 4 illustrates repeated shear wave acquisition sequences (a) and the geometric structure (b) of an ultrasound wave device according to one embodiment of the present invention.
FIG. 5 is a diagram illustrating the flow and data of a shear-wave speed measurement method according to one embodiment of the present invention.
FIG. 6 illustrates multiple shear wave data (a) and (b) acquired by a multiple pushing + multi-line detecting method and a graph (c) showing the superposition of shear-wave speed data according to one embodiment of the present invention.
FIG. 7 is a diagram illustrating the configuration of an ultrasound wave device according to one embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present invention, and these terms may be varied according to the intent or custom of a user or an operator. Therefore, the definitions of the terms used herein should follow contexts disclosed herein.

Hereinafter, embodiments of the present invention will be described in detail with reference to the accompanying drawings. However, the present invention may be realized in various forms, and the scope of the present invention is not limited to such embodiments. The embodiments of the present invention are provided to aid those skilled in the art in the explanation and the understanding of the present invention.

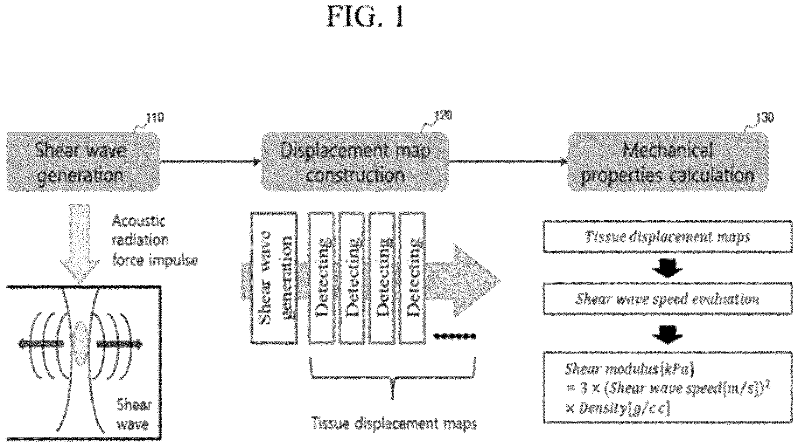

FIG. 1 is a diagram illustrating the flow of a conventional shear wave parameter measurement method.

An ultrasound wave device generates a shear wave by successively pushing a beam ultrasound signal (hereinafter, referred to as a "push beam") to push a region of interest (ROI) of an object through an ultrasound probe (110).

The ultrasound wave device pushes a push beam pulse to the ROI of the object using the channels of the ultrasound probe. The ultrasound wave device may push a focused push beam to the ROI of the object. In this case, a shear wave is generated in the ROI by the push beam. For example, a shear wave may be generated around the region pushed by an acoustic radiation force impulse (ARFI). When a shear wave is directly generated by the pulse of the push beam, the push beam is referred to as ARFI. A shear wave propagates in a direction that is substantially orthogonal to the direction of ARFI. A shear wave is a form of displacement.

Subsequently, the ultrasound wave device acquires time-series data in the detection area and converts it into a tissue displacement map (120).

Next, the ultrasound wave device calculates the mechanical parameter of the shear wave that is proportional to tissue stiffness using the tissue displacement map (130). For example, the ultrasound wave device calculates the shear-wave speed and estimates the shear modulus of the shear wave. Since the shear-wave speed (for example, 1-10 m/s) is much slower than the average speed (i.e., 1540 m/s) of ultrasound signals within the object, the ultrasound wave device may use an ultrasound signal (hereinafter, referred to as a "detection ultrasound wave") for detecting a shear wave. For instance, the ultrasound wave device may calculate the mechanical parameter (e.g., speed) of a shear wave by transmitting a detection ultrasound wave while the shear wave is propagating.

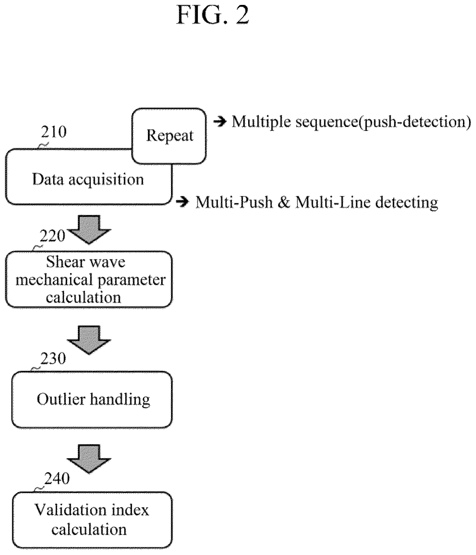

FIG. 2 is a diagram illustrating the flow of a method of acquiring a shear wave parameter according to one embodiment of the present invention.

Referring to FIG. 2, an ultrasound wave device acquires multiple shear wave data by repeating multiple shear wave acquisition sequences (210). The multiple shear wave acquisition sequences consist of push-detection operations, involving pushing multiple ARFIs and detecting data from multiple detecting lines. For example, the multiple shear wave acquisition sequences include a first sequence and a second sequence, wherein the first sequence comprises operations of performing first ARFI pushing to generate a shear wave in an ROI and detecting the shear wave generated by the first ARFI pushing from the multiple detecting lines and the second sequence comprises operations of performing second ARFI pushing to generate a shear wave in the ROI and detecting the shear wave generated by the second ARFI pushing from the multiple detecting lines. Since this method pushes multiple ARFIs and detects shear wave data from multiple detecting lines (multi-line detecting), it is referred to as a "multiple pushing + multi-line detecting method."

Subsequently, the ultrasound wave device calculates the mechanical parameter of the acquired shear wave (220). In this case, the ultrasound wave device may measure the particle velocity of the shear wave, the lag of the shear wave, the speed of the shear wave, and the elastic modulus of the shear wave.

Next, the ultrasound wave device determines an inlier of shear wave data and handles outliers (230). In this case, the ultrasound wave device may superimpose the multiple shear wave data based on shear-wave speed and determine an inlier of each shear wave data for each depth when superimposed. For example, when both first shear-wave speed data and second shear-wave speed data at the same depth are outliers, the ultrasound wave device determines that the corresponding depth is an invalid position. In contrast, when at least one of the first shear-wave speed data or the second shear-wave speed data at the same depth is an inlier, the ultrasound wave device determines that the corresponding depth is a valid position.

Subsequently, the ultrasound wave device calculates a validation index using the valid position of the shear-wave speed data (240).

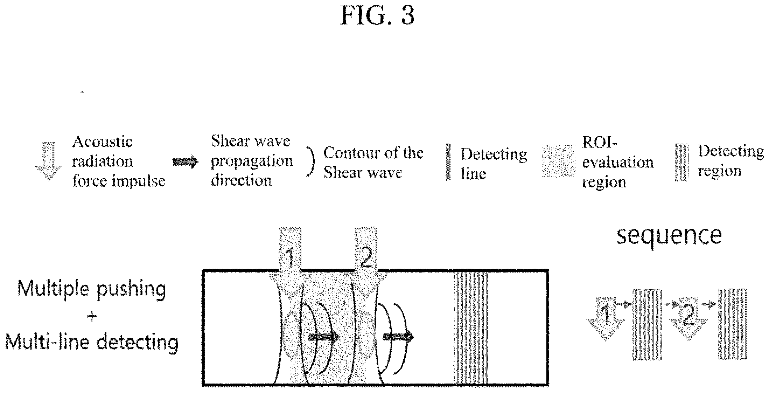

FIG. 3 is a diagram illustrating a multiple pushing + multi-line detecting method according to one embodiment of the present invention.

Referring to FIG. 3, when the ultrasound wave device pushes ARFI to an ROI-evaluation region, a shear wave propagates, and shear wave data is detected from a detecting region. In this case, there are multiple ARFI, and the detecting region includes multiple detecting lines. The sequence consists of first ARFI pushing, detecting shear wave data from multiple detecting lines, second ARFI pushing, and detecting shear wave data from multiple detecting lines. The multiple pushing + multi-line detecting method is not affected by beam spacing and does not exhibit shearing phenomenon. The arrival times of the shear waves are the same along the detecting lines. Therefore, the validity of detection in the detecting region may be determined, and result values may be selectively derived to improve validation.

In contrast, single pushing + multi-line detecting method, which involves pushing a single ARFI, is influenced by beam spacing, exhibits high measurement variance, and shows significant difference in arrival time along the detecting lines. Therefore, depending on the measurement depth, correlation coefficient and shear modulus errors occur and shearing phenomenon takes place.

Furthermore, in the multiple pushing + single line detecting method, which detects shear wave data from a single detecting line, there is a region where shear wave data detection is impossible if the detecting line is positioned in a region where detection is challenging. For example, even if the ROI is located at a position suitable for measurement (where the influence of heartbeat and respiration is minimal), the measurement results are affected by the location of the detecting region. When the measurement results are unstable, it is difficult to determine the factors causing it.

In comparison, the multiple pushing + multi-line detecting method improves the validation and accuracy of measurements by diversifying the detecting region positions and acquiring multiple data, making it easier to identify outliers and determine data validity.

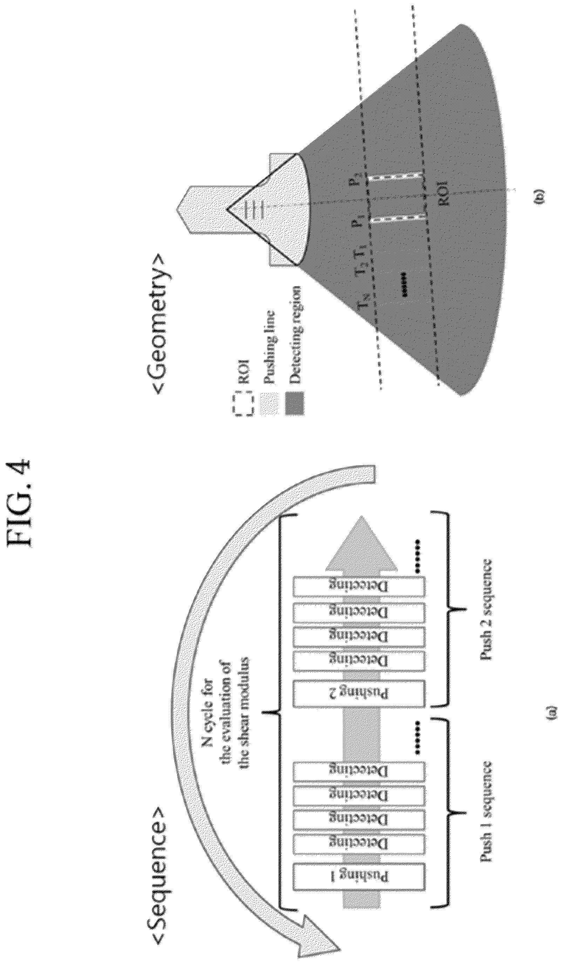

FIG. 4 illustrates repeated shear wave acquisition sequences (a) and the geometric structure (b) of an ultrasound wave device according to one embodiment of the present invention.

Referring to FIG. 4, the repeated shear wave acquisition sequences include, for example, a first pushing sequence (first ARFI pushing (P1) -> detecting shear wave data from a first detecting line (T1)) and a second pushing sequence (second ARFI pushing (P2) -> detecting shear wave data from a second detecting line (T2)). The ultrasound wave device repeatedly performs the shear wave acquisition sequences. Preferably, there may be three or more repeated shear wave acquisition sequences. This method is referred to as a "multiple-sequence method," where multiple shear wave acquisition sequences are employed and shear wave data is detected from a single detecting line with its location varying in each shear wave acquisition sequence. The multiple-sequence method acquires shear-wave speed samples by using data acquired through repetition of shear wave acquisition sequences, allowing the number of shear-wave speed samples to increase at each depth, and efficient handling of outliers can be achieved by determining an inlier of data. In addition, data validity can be improved by minimizing the detecting locations of shear wave data and motion artifact variables.

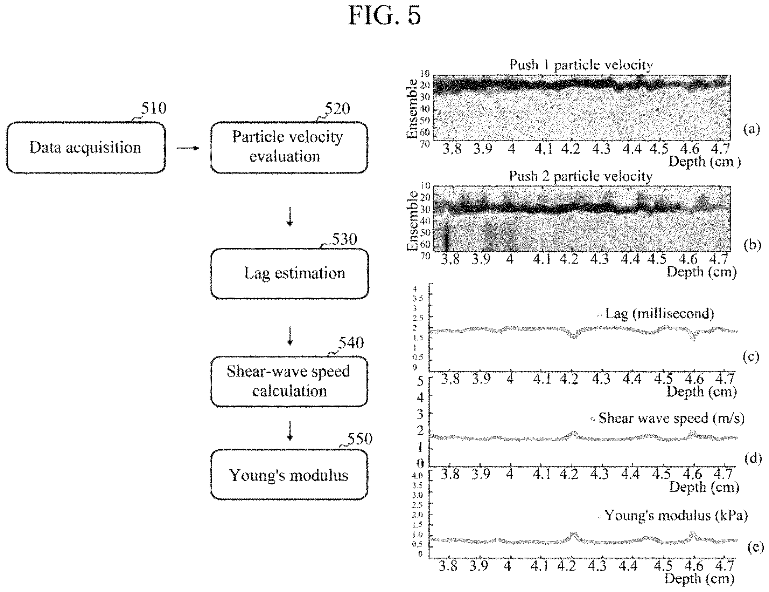

FIG. 5 is a diagram illustrating the flow and data of a shear-wave speed measurement method according to one embodiment of the present invention.

Referring to FIG. 5, an ultrasound wave device may acquire multiple shear wave data using the multiple pushing + multi-line detecting method (510) and measure the particle velocities of multiple shear waves (520). Here, auto-correlation between multiple shear wave data may be used.

Next, the ultrasound wave device estimates a shear-wave lag (530). Here, auto-correlation between multiple shear wave data may be used.

Subsequently, the ultrasound wave device calculates a shear-wave speed (540). The shear-wave speed [m/s] can be calculated as the pushing distance [m] divided by the lag [sec].

Then, the ultrasound wave device estimates the shear wave elastic modulus. In this case, the shear wave elastic modulus [kPa] may be calculated as *3×(shear wave speed) [m*/*s²]×density [g*/*cc].*

(a) and (b) in FIG. 5 depict the particle velocities (Push 1 particle velocity and Push 2 particle velocity) of the shear wave data acquired by the multiple pushing + multi-line detecting method, (c) represents the shear wave lag, (d) shows the shear-wave speed, and (e) depicts the shear wave elastic modulus.

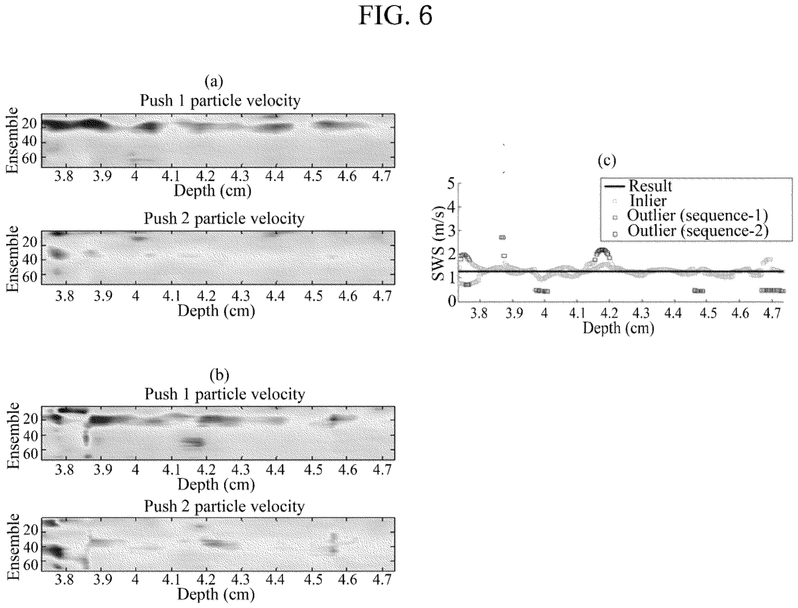

FIG. 6 illustrates multiple shear wave data (a) and (b) acquired by a multiple pushing + multi-line detecting method and a graph (c) showing the superposition of shear-wave speed data according to one embodiment of the present invention.

Referring to FIG. 6, the ultrasound wave device acquires first data (a) and second data (b), and each data includes first shear-wave speed data (Push 1 particle velocity data) and second shear-wave speed data (Push 2 particle velocity data).

The ultrasound wave device superimposes multiple shear-wave speed data based on the shear-wave speed and determines the inlier of each shear-wave speed data for each depth when superimposed. For example, if both the first shear-wave speed data and second shear-wave speed data at the same depth are outliers, the ultrasound wave device determines that the corresponding depth is an invalid position, and when at least one of the first shear-wave speed data or the second shear-wave speed data at the same depth is an inlier, the ultrasound wave device determines that the corresponding depth is a valid position.

Referring to (c) in FIG. 6, the result of data superposition includes inliers and outliers (outlier-sequence 1 and outlier-sequence 2). The ultrasound wave device may improve the accuracy and validation of the data by complementing the valid regions for each data.

And, the ultrasound wave device superimposes shear-wave speed data 1 (sequence 1) and shear-wave speed data 2 (sequence 2) and then determines the validity of data at each depth. Data results include inliers and outliers (outlier-sequence1, outlier-sequence 2). The superimposition of multiple data allows the number of inliers and valid regions to increase, thereby improving the accuracy and validation of the data.

In another example, the ultrasound wave device excludes outliers, which are data where the propagation of the shear wave generated by ARFI pushing is not successfully detected. For this purpose, after measuring the shear wave particle velocity, the ultrasound wave device calculates the signal-to-noise ratio (SNR) of the shear-wave speed data. In this case, a shear wave SNR below a predetermined value is determined to be invalid and processed as an outlier.

The ultrasound wave device may calculate a validation index using the valid positions of the shear-wave speed data. The ultrasound wave device uses the proportion of the region containing inliers used in the results to the measurement area as the validation index. For example, the validation index is calculated as the number of inlier depth positions divided by the number of total axial positions. The higher the number of inliers per depth, the higher the accuracy and validation of the data.

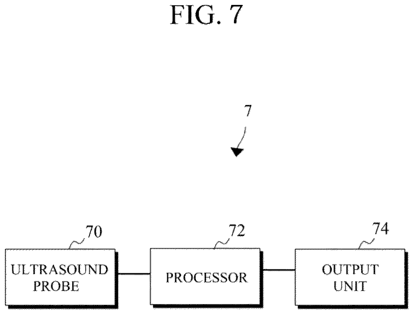

FIG. 7 is a diagram illustrating the configuration of an ultrasound wave device according to one embodiment of the present invention.

Referring to FIG. 7, the ultrasound wave device 7 includes an ultrasound probe 70, a processor 72, and an output unit 74.

The ultrasound probe 70 emits an ultrasound signal to an object according to a driving signal applied from an ultrasound transmitting unit and receives an echo signal reflected from the object. The ultrasound probe 70 includes multiple transducers. The multiple transducers vibrate in response to a transmitted electrical signal, generating ultrasound, which is acoustic energy. Additionally, the ultrasound probe 70 may be connected to the main body of the ultrasound wave device in a wired or wireless manner, and the ultrasound wave device may include multiple ultrasound probes 70 depending on the implementation.

The ultrasound probe 70 may induce a shear wave by pushing multiple ARFIs into an ROI of the object. Subsequently, the ultrasound probe 70 may transmit multiple detection ultrasound waves to the object to track the shear wave and may acquire multiple shear wave data by receiving from the object a response signal for each detection ultrasound wave. In this case, the ultrasound probe 70 transmits the detection ultrasound waves to a detecting region that includes multiple detecting lines, and acquires shear wave data by receiving response signals from the detecting region.

The processor 72 may perform signal processing on the response signals received from the ultrasound probe 70. For example, the processor 72 may process an echo signal received from the ultrasound probe 70 for signal processing to generate shear wave data, and may include an amplifier, an analog-to-digital converter (ADC), a reception delay unit, and a summation unit.

The processor 72 according to one embodiment calculates a mechanical parameter of the shear wave data based on the shear wave data received through the ultrasound probe 70 and generates an elastography image of the object.

The output unit 74 displays the image processing results, including the generated elastography image, on the screen.

Heretofore, the present invention has been described by focusing on the exemplary embodiments. It can be understood by those skilled in the art to which the present invention pertains that the present invention can be implemented in modified forms without departing from the essential feature of the present invention. Therefore, the disclosed embodiments should be considered as illustrative rather than determinative. The scope of the present invention is defined by the appended claims rather than by the foregoing description, and all differences within the scope of equivalents thereof should be construed as being included in the present invention.

## Claims

1. A shear wave parameter measurement method using an ultrasound wave device, the shear wave parameter measurement method comprising the steps, performed by the ultrasound wave device, of:
acquiring multiple shear wave data by repeating multiple shear wave acquisition sequences; and
calculating mechanical parameters of the acquired multiple shear wave data,
wherein the multiple shear wave acquisition sequences comprise a first sequence and a second sequence,
the first sequence comprises operations of performing first acoustic radiation force impulse (ARFI) pushing to generate a shear wave in a region of interest and detecting, from multiple detecting lines, the shear wave generated by the first ARFI pushing, and
the second sequence includes operations of performing second ARFI pushing to generate a shear wave in the region of interest and detecting, from the multiple detecting lines, the shear wave generated by the second ARFI pushing.

2. The shear wave parameter measurement method of claim 1, wherein the calculating of the mechanical parameter of the multiple shear wave data comprises:
measuring a particle velocity of each shear wave;
estimating a lag of each shear wave;
calculating a speed of each shear wave; and
estimating an elastic modulus of each shear wave.

3. The shear wave parameter measurement method of claim 1, further comprising the steps of:
superimposing multiple shear-wave speed data;
determining an inlier of each shear-wave speed data for each depth when superimposed, and handling outliers.

4. The shear wave parameter measurement method of claim 3, wherein in the determining of the inlier of each shear-wave speed data, when both first shear-wave speed data and second shear-wave speed data at the same depth are outliers, the corresponding depth is determined to be an invalid position.

5. The shear wave parameter measurement method of claim 3, wherein in the determining of the inlier of each shear-wave speed data, when at least one of first shear-wave speed data or second shear-wave speed data at the same depth is an inlier, the corresponding depth is determined to be a valid position.

6. The shear wave parameter measurement method of claim 5, further comprising the step of calculating a validation index using the valid position of the shear-wave speed data.

7. The shear wave parameter measurement method of claim 1, further comprising the steps of:
measuring a signal-to-noise ratio (SNR) of each shear-wave speed data;
determining an inlier of each shear-wave speed data using the SNR of each shear-wave speed energy; and
handling outliers.

8. A shear wave parameter measurement method using an ultrasound wave device, the shear wave parameter measurement method comprising the steps, performed by the ultrasound wave device, of:
acquiring multiple shear wave data by repeating multiple shear wave acquisition sequences; and
calculating a mechanical parameter of the acquired multiple shear wave data,
wherein the multiple shear wave acquisition sequences comprise a first sequence and a second sequence,
the first sequence comprises operations of performing acoustic radiation force impulse (ARFI) pushing to generate a shear wave in a region of interest and detecting, from a first detecting line, the shear wave generated by the first ARFI pushing, and
the second sequence comprises operations of performing second ARFI pushing to generate a shear wave in a region of interest and detecting, from a second detecting line, the shear wave generated by the second ARFI pushing.

9. The shear wave parameter measurement method of claim 8, further comprising the steps of:
superimposing multiple shear-wave speed data;
determining the inlier of each shear-wave speed data for each depth when superimposed; and
handling outliers.

10. An ultrasound wave device comprising:
an ultrasound probe configured to acquire multiple shear wave data by repeating multiple shear wave acquisition sequences; and
a processor configured to calculate a mechanical parameter of the shear wave data acquired through the ultrasound probe,
wherein the multiple shear wave acquisition sequences comprise a first sequence and a second sequence,
the first sequence comprises operations of performing first acoustic radiation force impulse (ARFI) pushing to generate a shear wave in a region of interest and detecting, from multiple detecting lines, the shear wave generated by the first ARFI pushing, and
the second sequence comprises operations of performing second ARFI pushing to generate a shear wave in a region of interest and detecting, from the multiple detecting lines, the shear wave generated by the second ARFI pushing.
